(19) Europäisches Patentamt
European Patent Office
Office européen des brevets

(11) EP 3 277 728 B1

(12) **EUROPEAN PATENT SPECIFICATION**

(45) Date of publication and mention of the grant of the patent:
**04.10.2023 Bulletin 2023/40**

(21) Application number: **16715711.4**

(22) Date of filing: **28.03.2016**

(51) International Patent Classification (IPC):
***C08B 11/14*** *(2006.01)* ***A61K 9/00*** *(2006.01)*
***C08B 11/193*** *(2006.01)* ***C08L 1/28*** *(2006.01)*

(52) Cooperative Patent Classification (CPC):
**A61K 47/38; A61K 9/0043; C08B 11/14; C08B 11/193; C08L 1/288**

(86) International application number:
**PCT/US2016/024491**

(87) International publication number:
**WO 2016/160688 (06.10.2016 Gazette 2016/40)**

(54) **COMPOSITION CONTAINING CATIONIC HYDROXYETHYL CELLULOSE**
ZUSAMMENSETZUNG MIT KATIONISCHER HYDROXYETHYLCELLULOSE
COMPOSITION CONTENANT DE L'HYDROXYÉTHYLCELLULOSE CATIONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.04.2015 US 201562142045 P**

(43) Date of publication of application:
**07.02.2018 Bulletin 2018/06**

(73) Proprietors:
- **Nutrition & Biosciences USA 1, LLC Rochester, NY 14623 (US)**
- **Rohm and Haas Company Philadelphia, PA 19106 (US)**

(72) Inventors:
- **KATZ, Joshua S. Collegeville, PA 19426 (US)**
- **TAN, Yujing Midland, MI 48667 (US)**
- **PARTAIN, Emmett M. Collegeville, PA 19426 (US)**
- **DIELMAN, Demetrius Midland, MI 48667 (US)**
- **JORDAN, Susan L. Collegeville, PA 19426 (US)**
- **CURTIS-FISK, Jaime L. Midland, MI 48667 (US)**
- **DEPUIT, Ryan J. Freeport, TX 77541 (US)**

(74) Representative: **International N&H EMEA Parallelvej 16 2800 Kongens Lyngby (DK)**

(56) References cited:
**EP-A2- 0 189 935**
**WO-A1-2008/042635**
**US-A- 5 407 919**
**US-A- 5 645 827**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 277 728 B1

## Description

Mucosal surfaces line various cavities in a living body, including those exposed to the external atmosphere. Mucosal surfaces are involved in absorption of compounds into the body. Consequently a useful method of introducing a physiologically active agent into the body is to apply a composition containing the physiologically active agent to the mucosal surface. When applying such a composition to a mucosal surface, it is desirable that the composition reside on the mucosal surface for a relatively long time. It is also desirable that the composition allow the physiologically active agent to readily permeate through the mucosal surface so that the physiologically active agent can enter the tissues and/or the bloodstream of the living body. Often, a composition that contains a physiologically active agent also contains one or more additional compound called "excipients." It is desirable that an excipient improve the residence time of the composition and/or improves the permeation of the physiologically active agent through the mucosal surface. An important mucosal surface in the human body for introduction of physiologically active agents is the mucosal surface inside the nasal cavity.

US 3,472,840 describes certain specific cationic polysaccharide polymers. It is desired to provide cationic polysaccharide polymers that provide improved permeation through mucosal surfaces compared to those described by US 3,472,840. In the past, chitosan has been used as an excipient. Chitosan is derived from a natural product and therefore is subject to variability in quality and characteristics; also, the usefulness of chitosan is undesirably limited because chitosan is not soluble at certain desirable pH values. It is desired to provide a synthetic polymer that performs well as an excipient and/or that is soluble over a desirably wide range of pH values.

The following is a statement of the invention.

The first aspect of the present invention is an aqueous solution for use in a method of delivering a drug into the tissues and/or bloodstream of a living body by permeation through a mucosal surface in a nasal cavity, comprising:

(a) a cationic polymer dissolved in water, wherein said cationic polymer comprises a hydrophobic quaternary ammonium group covalently attached to a hydroxyethyl cellulose polymer backbone; wherein the cationic polymer has a mole ratio of non-hydrophobic quaternary ammonium groups to hydrophobic quaternary ammonium groups of 0:1; and

(b) one or more physiologically active agents

In a second aspect is disclosed herein a method of delivering a drug to a mucosal surface in a living body, said method comprising applying the aqueous solution of the first aspect to said mucosal surface, wherein said aqueous solution of the first aspect comprises said drug.

The following is a detailed description of the invention.

As used herein, the following terms have the designated definitions, unless the context clearly indicates otherwise.

Mucosal surfaces are found in living bodies of animals and humans. Mucosal surfaces contain epithelium, which produces and excretes mucus. Examples of mucosal surfaces are found in the nasal cavity, the mouth, the eye, the ear, the vagina, the esophagus, the stomach, the intestines, and other parts of the body.

A compound is considered herein to be cationic if an atom or a chemical group that bears a positive charge is covalently bound to the compound. A cationic functional group is an atom or a chemical group that bears a positive charge. The cationic functional group bears a positive charge at all pH values over a range that includes the range of 4 to 11.

An amount of polymer is considered herein to be dissolved in water if the mixture of that amount of the polymer and water forms a composition that at 25°C is homogeneous and that does not show phase separation of the polymer from the water at 25°C.

As used herein, a drug is a compound having beneficial prophylactic and/or therapeutic properties when administered to an individual, typically a mammal, especially a human individual.

As used herein, a hydrophobic group is a chemical group that contains a group of 8 or more carbon atoms, where the carbon atoms are connected to each other in a manner that is linear, cyclic, branched, or a combination thereof, and where the only atoms in the hydrophobic group are carbon and hydrogen.

As used herein, a hydrophobic quaternary ammonium group is a chemical group having the structure I

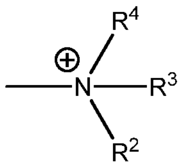

I

where $-R^2$ and $-R^3$ are substituted or unsubstituted hydrocarbon groups each containing one or more carbon atom, and $-R^4$ contains one or more hydrophobic group. A non-hydrophobic quaternary ammonium group is a chemical group having structure I in which none of $-R^2$, $-R^3$, and $-R^4$ contains a hydrophobic group.

The present invention involves a cationic polymer that contains a cationic functional group attached to a hydroxyethyl cellulose polymer backbone. That is, the cationic polymer has a structure that would result if a molecule of the hydroxyethyl cellulose polymer (the "backbone" polymer) were subjected to one or more chemical reactions to replace one or more of the hydroxyl groups on the hydroxyethyl cellulose polymer with cationic functional groups. Regardless of the method of making the cationic polymer, the cationic polymer can be characterized by the properties of the backbone polymer.

Hydroxyethyl cellulose (HEC) polymer has repeat units of the structure II:

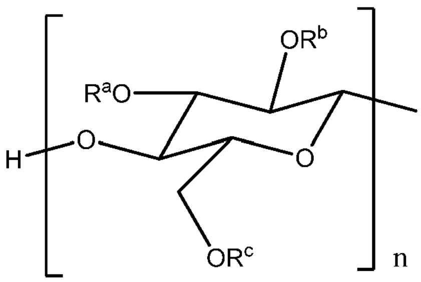

II

In structure II, the repeat unit is shown within the brackets. The degree of polymerization (n) is 10 or higher and is sufficiently large that structure II is a polymer; that is, when n is large enough, the 2% standard solution viscosity (as defined below) of the HEC will be 10 mPa*s or higher. $-R^a$, $-R^b$, and $-R^c$ is each $-[CH_2CH_2O]_X-H$, where each x is chosen from 0, 1, 2, 3, or 4. The choice of $-R^a$, $-R^b$, and $-R^c$ may be the same in each repeat unit, or different repeat units may have different choices of $-R^a$, $-R^b$, and $-R^c$. One or more repeat units has one or more of $-R^a$, $-R^b$, and $-R^c$ in which x is from 1 to 4.

The cationic polymer of the present invention has repeat units of the structure II in which one or more of $-R^a$, $-R^b$, and $-R^c$ has the structure $-[CH_2CH_2O]_x-R^1$, where $R^1$ has structure III:

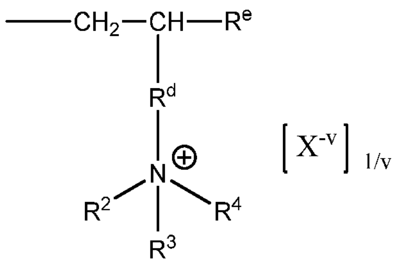

III

where $-R^d-$ is a bivalent organic group, and $-R^e$ is either a hydrogen atom or a hydroxyl group. Preferably, $-R^d-$ is a hydrocarbon group with 0 to 8 carbon atoms; more preferably with 1 to 2 carbon atoms; more preferably with 1 carbon atom. Preferably, $-R^e$ is a hydroxyl group. $-R^2$, $-R^3$, and $-R^4$ is each independently a substituted or unsubstituted hydrocarbon group. Preferably $-R^2$ and $-R^3$ are unsubstituted hydrocarbon groups; more preferably $-R^2$ and $-R^3$ are alkyl groups. Preferably $-R^2$ and $-R^3$, is each independently an alkyl group with 3 or fewer carbon atoms; more preferably and alkyl group with 2 or fewer carbon atoms; more preferably a methyl group. $-R^4$ is a chemical group containing a hydrophobic group. Preferably, $-R^4$ is an alkyl group. Preferably, $-R^4$ has 10 or more carbon atoms; more preferably 12 or more carbon atoms. Preferably, $-R^4$ has 18 or fewer carbon atoms; more preferably 16 or fewer carbon atoms; more preferably 14 or fewer carbon atoms; more preferably 12 or fewer carbon atoms. Preferably, one or more $-R^b$ or $-R^c$ group has structure II. $X^{-v}$ is an anion of valence v. It is contemplated that if v is greater than 1, then v groups of structure III will be associated with each anion of valence v. Preferred anions are halide ions; more preferred is chloride ion.

The cationic polymer of the present invention has a mole ratio of non-hydrophobic quaternary ammonium groups to hydrophobic quaternary ammonium groups is 0:1.

The cationic polymer of the present invention may be characterized by the viscosity of a solution of either 1% or 2% by weight of the cationic polymer in water at 25°C. That viscosity is measured at 25.0°C and a shear rate of 6.31 $sec^{-1}$ using a TA Instruments DHR-3 rheometer equipped with either a stainless steel cone and plate sensor (60 mm diameter and 0.5° cone angle) or a concentric cylinder cup and bob sensor. If the viscosity of the 2% solution is greater than 18,000 mPa*s, then a solution of 1% by weight of the cationic polymer in water is made and tested by the same

method at 25°C. The result of this viscosity testing is reported herein as the "standard solution viscosity."

Preferably, the standard solution viscosity of the cationic polymer of the present invention in a 2% by weight solution is 50 mPa*s or higher; more preferably 100 mPa*s or higher. Preferably, the standard solution viscosity of the cationic polymer of the present invention in a 1% by weight solution is 30,000 mPa*s or lower. Preferably, the standard solution viscosity of the cationic polymer of the present invention in a 2% by weight solution is 18,000 mPa*s or lower; more preferably 10,000 mPa*s or lower.

The cationic polymer of the present invention may be characterized by the weightaverage molecular weight (Mw), which is measured by gel permeation chromatography. Preferably, Mw is 50,000 or higher; more preferably 100,000 or higher; more preferably 200,000 or higher. Preferably, Mw is 1,000,000 or lower; more preferably 500,000 or lower.

The cationic polymer of the present invention may be characterized by the cationic degree of substitution (CDS), which is defined as the molar ratio of repeat anhydroglucose units with the structure III over total repeat anhydroglucose units. The CDS is measured and calculated from Kjeldahl nitrogen analysis. Preferably, the cationic degree of substitution is 0.01 or higher; more preferably 0.02 or higher; more preferably 0.05 or higher.

A preferred method of making the cationic polymer is to react a hydroxyethyl cellulose polymer with a compound of structure IV, V, or VI:

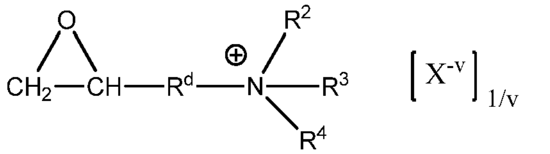

IV

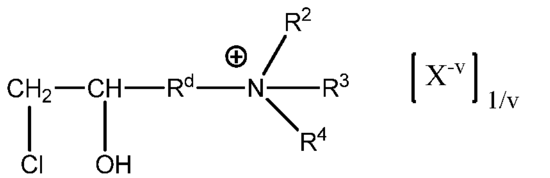

V

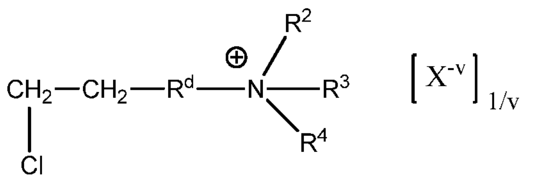

VI

where the $R^d$, $R^2$, $R^3$, $R^4$, X, and v are defined above.

The present invention involves a solution that contains a cationic polymer dissolved in water. Preferably, the amount of water in the solution, by weight based on the total weight of the volatile components in the solution, is 50% or more; more preferably 75% or more; more preferably 90% or more.

The amount of the cationic polymer in the solution is preferably, by weight based on the weight of the solution, 0.01% or more; more preferably 0.1% or more. The amount of polymer in the solution is preferably, by weight based on the weight of the solution, 10% or less; 5% or less; more preferably 2% or less; more preferably 1% or less.

The solution optionally contains additional ingredients such as, for example, surfactants, thickeners, buffers, pH adjusters, preservatives, and mixtures thereof.

Preferably the solution is a buffer solution that contains inorganic salts. One preferred buffer solution is phosphate buffered saline (PBS) solution, which contains sodium chloride and a sodium salt of a phosphorous-containing anion, and optionally also contains potassium chloride and a potassium salt of a phosphorous-containing anion.

The solution may be a liquid, a gel, a lotion, a cream, or another form. Preferred is a liquid. Preferably the viscosity of the solution, as measured by steady shear viscometry using cone and plate at 10 $sec^{-1}$ at 25°C, is 1,000 mPa-s or less; more preferably 300 mPa-s or less; more preferably 100 mPa-s or less; more preferably 30 mPa-s or less; more preferably 10 mPa-s or less.

Mucosal surfaces disclosed herein are the mucosal surfaces of the nasal cavity, the mouth, the eye, the ear, the vagina, the esophagus, the stomach, the intestines, and combinations thereof; The mucosal surfaces of the nasal cavity is used for delivering a drug into the tissues and/or bloodstream of a living body by permeation of aqueous solution according to the present invention.

Preferably the composition of the present invention contains one or more physiologically active agents, preferably one or more physiologically active agents selected from the following: one or more drugs; one or more diagnostic agents; or one or more essential oils; or one or more physiologically active agents that are useful for cosmetic or nutritional purposes. Preferred physiologically active agents are drugs. Preferred drugs are soluble or dispersible in water at 15°C to 40°C, in concentrations that are therapeutically useful. Preferred drugs that, in the absence of an effective excipient, have undesirably low capability of absorption into the body through a mucosal surface.

Physiologically active agents that are useful for intranasal delivery are known in the art. Some physiologically active agents and some methods of intranasal delivery are described in WO 2015/009799.

The composition of the present invention is particularly useful for intranasal delivery of one or more physiologically active agents or for delivery through a mucosal membrane located in the nasal cavity, such as drugs utilized in therapies for allergic rhinitis, nasal congestion and infections, in treatments of diabetes, migraine, nausea, smoking cessation, acute pain relief, nocturnal enuresis, osteoporosis, vitamin B-12 deficiency, and for administering intranasal vaccine such as, for example, influenza vaccine; however, the physiologically active agents are not limited to these examples. Especially preferred drugs are acetaminophen, azelastine hydrochloride, beclomethasone dipropionate monohydrate, sumatriptan succinate (SS), dihydroergotamine mesylate, fluticasone propionate, triamcinolone acetonide, budesonide, fentanyl citrate, butorphanol tartrate, zolmitriptan, desmopressin acetate hydrate, salmon calcitonin, nafarelin acetate, buserelin acetate, elcatonin, oxytocin, insulin, mometasone furoate, estradiol, metoclopramide, xylometazoline hydrochloride, ipratropium bromide hydrate, olopatadine hydrochloride, oxymetazoline hydrochloride, dexpanthenol, hydrocortisone, naphazoline hydrochloride, phenylephrine hydrochloride, mepyramine maleate, phenylephrine hydrochloride, cromolyn sodium, levocabastine hydrochloride, vitamin B12, prednisolone sodium metasulphobenzoate, naphazoline nitrate, tetrahydrozoline hydrochloride, chlorpheniramine maleate, benzethonium chloride, ketotifen fumarate, histamine dihydrochloride, fusafungine, or combinations thereof. Examples of essential oils are menthol, methyl salicylate, thymol, eucalyptus oil, camphor, anise, sweet orange, or combinations thereof.

The following are examples of the present invention. Example numbers starting with "C" denote comparative examples.

The following comparative polymers were tested:

Comp1 = SOFTCAT™ SX-1300H, from the Dow Chemical Company, contains both hydrophobic and non-hydrophobic cationic groups. The mole ratio of non-hydrophobic cationic groups to hydrophobic cationic groups is larger than 5:1.
Comp2 = UCARE™ KG-30M, quatemized HEC, from the Dow Chemical Company; has no hydrophobic group.
Comp3 = UCARE™ JR-400 polymer, cationic HEC from the Dow Chemical Company; has no hydrophobic group.
Chitosan = naturally-derived amine-functional polymer; does not have hydrophobic groups.

The following abbreviations are used.

TEER = trans-endothelial electrical resistance
API = active pharmaceutical ingredient, a type of physiologically active agent
SS = sumatriptan succinate (a drug)
PBS = phosphate buffer saline solution

The following Example polymers were used. Each Example polymer is described by structure II above, where $-R^a$ and $-R^b$ are -H; where $-R^c$ is $-[CH_2CH_2O]_x-R^1$, where some repeat units have x=1 or 2; where $-R^1$ has structure III above, where $-R^d$ is $-CH_2-$, where $-R^2$ and $-R^3$ are methyl, and where $-R^4$ is an alkyl hydrophobic group. The Example polymers were as follows:

Example Polymers

| Polymer | MW | CDS(3) | Viscosity(4) | %(5) | Phobe(6) |
|---|---|---|---|---|---|
| P1 | 275,000 | 0.0784 | 8617 | 2 | 12 |
| P2 | 275,000 | 0.0191 | 8833 | 2 | 18 |
| P3 | 1,000,000 | 0.131 | 6719 | 2 | 12 |
| P4 | 1,600,000 | 0.0257 | 6035 | 1 | 18 |
| P5 | 1,600,000 | 0.079 | 17058 | 2 | 12 |

(continued)

| Polymer | MW | CDS(3) | Viscosity(4) | %(5) | Phobe(6) |
|---|---|---|---|---|---|
| P6 | 1,000,000 | 0.075 | 11634 | 1 | 12 |
| P7 | 280,000 | 0.078 | 207 | 2 | 12 |
| P8 | 1,600,000 | 0.14 | 6400 | 1 | 12 |
| (3) Cationic degree of substitution<br>(4) viscosity of solution in water at 25°C, mPa*s<br>(5) concentration of polymer in viscosity test solution, weight %<br>(6) number of carbon atoms in $-R^4$ | | | | | |

EpiAirway™ Tissue Models, 0.2% TRITON™ X-100 surfactant, and 3-(4,5-dimethylthiazol-2-yl)-2,5-diphenyltetrazolium bromide (MTT) assays were acquired from MatTek Corporation. After receiving tissues (24), they were removed from the agar media, moved to clean 6-well plates containing 0.9 mL of fresh Assay Media, and cultured overnight (eighteen hours) in a sterile environment according to the product information. The Assay Media was composed by base medium (Dulbecco's Modified Eagle's Medium), growth factors/hormones (epidermal growth factor, insulin, hydrocortisone and other stimulators of epidermal differentiation), antibiotics (gentamicin 5 μg/mL) and anti-fungal agent (amphotericin B 0.25 μg/mL).

Tissues were removed from the incubator (37 °C, 5 % $CO_2$) and prepared for a media exchange. Media exchange was performed on all tissues before being returned to the incubator. After 1-2 hours of additional incubation, twelve tissues were removed from the incubator, media was discarded, and tissues were rinsed with Phosphate Buffer Saline (PBS) (from Dulbecco) and tested for TEER before being used for time dependent studies of permeation of API). TEER measurement was performed using an Endohm chamber coupled with $EVOM^2$ ™ resistance meter from World Precision Instruments Company. Briefly, each tissue was placed into the Endohm chamber and covered by a cup with electrode, and reading shown on the resistance meter was recorded. Following TEER, tissues were moved to pre-labeled clean 24-well plates containing 250 μL of fresh media in each well (4 or 6 tissues per plate to allow for full permeation study). The remaining twelve tissues were left in the incubator for an additional 24 hours of incubation, to be used for additional permeation studies on the following day.

Donor solutions were prepared 16-20 hours prior to use. The donor solutions were prepared in the following manner: predetermined concentrations/amounts of excipient (surfactant or polymer) were analytically weighed into 50 mL conical tubes, diluted with an appropriate amount of PBS, placed on a rocking shaker for approximately 4 hours at room temperature (approximately 23°C) and allowed to hydrate. After hydration, the donor solutions were sterile filtered using Steriflip® filter units from Millipore and stored at 4°C until use.

All donor solutions had 2 mg/mL SS, except for Comparative Example 1, which used 50 mg/mL of SS.

Permeation studies were carried out in the following manner: donor solutions (100 μL) were carefully pipetted onto the apical surface of their respective tissues and incubated at 37°C, 5% $CO_2$ for 5 minutes (5 minute time point). After incubation, each tissue was moved to a new well with fresh media. Receiver solution from the previous well was collected and placed in a pre-labeled Waters Total Recovery LC/GC vial on dry ice. The tissues were then returned to the incubator for an additional 10 minute incubation period (15 minute time point). After incubation, the process of moving the tissues to the next well, collection of the permeated receiver solutions and incubation was repeated for additional time points up to 240 minutes. Following the 240 minute experimental period, the remaining donor solution on the apical surface of the tissue was collected and placed on dry ice, tissues were rinsed with PBS, and the final TEER measurements were taken.

Following the final TEER measurements, tissue percent viability was measured using the MTT Assay. This kit was used to indirectly measure the amount of nicotinamide adenine dinucleotide phosphate (NADPH) produced by the cells by measuring optical density of the formazan at 570 nm. A positive correlation of NADPH amount and cell viability is known. The cells treated with PBS buffer only were set to 100% which was used for normalization of all other samples. The result reported ("Viability %") is the quotient obtained by dividing the optical density at 570 nm for a sample by the optical density at 570 nm of the PBS buffer only, for the same optical path length.

Donor and receiver solution samples were removed from storage at -80 °C, thawed on ice, and analyzed using the HPLC method developed for Sumatriptan Succinate. Agilent 1100 serial binary gradient liquid chromatograph system was used. Details as following:

| | |
|---|---|
| Column: | Waters SunFire C18, 3.5 μm, 3.0 x 100 mm, Lot No 150331961 |
| UV Detector Wavelength: | 280 nm |

(continued)

| | |
|---|---|
| Eluent A: | 0.1% Trifluoroacetic acid in DI Water |
| Eluent B: | 0.1% Trifluoroacetic acid in 1-Propanol |
| Gradient: | 2% B to 100% B in 20 minutes, hold 100% B for 1 minute, 100% B to 2% B in 1 minute. |
| Post Run Time: | 5 minutes |
| Flow Rate: | 0.4 mL/min |
| Column | Temperature Setting: 27 °C |

From the solutions collected at the various times, the effective permeation coefficient (Peff) was calculated by

$$Peff = (TSS) / [(MA) * (DC) * (PT)]$$

where

TSS = total amount of SS in permeation (in units of mg)
MA = membrane area (equaled 0.6 $cm^2$)
DC = donor concentration (in units of $mg/cm^3$)
PT = permeation time (equaled 14400 sec)

Comparative Example 1: Permeation of sumatriptan succinate (SS) in PBS buffer with various comparative cationic polymers in the solution. Results were as follows:

Permeation of SS

| Example: | C1-1 | C1-2 | C1-3 | C1-4 | C1-5 | C1-6 | C1-7 |
|---|---|---|---|---|---|---|---|
| Polymer: | none | Comp1 | Comp1 | Comp2 | Comp3 | Comp3 | Chitosan |
| Conc(1) | 0 | 0.5% | 0.1% | 0.1% | 0.5% | 0.1% | 0.1% |
| N(2) | 2 | 3 | 2 | 3 | 2 | 2 | 2 |
| | | | | | | | |
| Remain(3) | 62.1 | 51.5 | 55.7 | 65.8 | 52.5 | 64.2 | 38.9 |
| Perm(4) | 2.0 | 2.2 | 1.8 | 2.3 | 1.4 | 1.8 | 37.6 |
| Peff(5) | 0.23 | 0.25 | 0.21 | 0.27 | 0.16 | 0.21 | 4.4 |

(1) % weight of polymer on total weight of solution
(2) number of replicate samples tested. Results shown are averages over the replicates
(3) weight % of SS remaining in donor solution based on total SS
(4) weight % or SS permeated through tissue based on total SS
(5) units are $10^{-6}$ cm/sec (for example, Peff of C1-1 was 0.23 X $10^{-6}$ cm/sec)

All of the synthetic polymers tested (Comparative Examples C1 through C6) showed far worse performance than Chitosan (Comparative Example C7).

Example 2: Permeation of SS using Example Polymer P7 on two different tissue samples. Permeation tests were performed as in Comparative Example 1. Results were as follows.

Permeation of SS

| Example: | C2-8 | C2-9 | 2-10 | 2-11 |
|---|---|---|---|---|
| Polymer: | none | Chitosan | P7 | P7 |
| Conc(1) | 0 | 0.1% | 0.5% | 0.5% |
| | | | | |
| Remain(3) | 102 | 63.5 | 31.9 | 53.6 |
| Perm(4) | 3 | 30.3 | 22.3 | 28.5 |
| Peff(5) | 0.35 | 3.5 | 2.6 | 3.3 |
| (1) % weight of polymer on total weight of solution<br>(3) weight % of SS remaining in donor solution based on total SS<br>(4) weight % or SS permeated through tissue based on total SS (5) units are $10^{-6}$ cm/sec | | | | |

Example polymer P7 performs far better than comparative polymers Comp1, Comp2, and Comp3. Also, P7 performs comparably to Chitosan.

Example 3: TEER Testing

TEER tests were performed on the sample reported in Example 2 above. Resistance drops from the initial value prior to permeation testing to the final value at the end of the permeation test; a larger drop indicates greater permeability. Results were as follows:

TEER tests

| Example: | C3-8 | C3-9 | 3-10 | 3-11 |
|---|---|---|---|---|
| Polymer: | none | Chitosan | P7 | P7 |
| Conc(1) | 0 | 0.1% | 0.5% | 0.5% |
| Resistance (ohms) | | | | |
| initial | 425 | 450 | 510 | 430 |
| final | 305 | 60 | 80 | 70 |
| (1) % weight of polymer on total weight of solution | | | | |

P7 and Chitosan show far greater drop in resistance (and therefore a greater tendency to assist permeation) than does the buffer solution alone.

Example 4: Tissue Viability Testing

After performing a permeation test as described above, tissues were given the viability test as described above. The samples contained SS. One sample had Triton™ X-100 surfactant at a level of 0.2 % by weight. Results were as follows:

Viability Test

| Example: | C4-8 | 4-10 | 4-12 |
|---|---|---|---|
| Excipient: | PBS | P7 | Surfactant |
| Conc(1) | 0 | 0.5% | 0.2% |

(continued)

| Example: | C4-8 | 4-10 | 4-12 |
|---|---|---|---|
| Viability (%) | 100 | 110 | 1 |
| (1) % weight of polymer on total weight of solution | | | |

The sample with surfactant had low viability. It is considered that the surfactant is likely to enhance permeation but cause a degradation of cell viability. The sample with P7 showed good permeability (as demonstrated above in a previous example) and good viability.

Example 5: Membrane recovery by the TEER method.

Samples were also tested for recovery in the TEER method. Samples had 2 mg/L SS. Results were as follows:

TEER Recovery test

| Example: | C5-12 | C5-13 | 5-14 |
|---|---|---|---|
| Excipient: | surfactant(6) | none | P7 |
| Conc(1) | 0.2% | 0 | 0.2% |
| Resistance (ohms) | | | |
| initial | 800 | 710 | 510 |
| at 4 hours permeation | 1 | 580 | 80 |
| 24 hours after permeation | 2 | 530 | 610 |
| (1) % weight of polymer on total weight of solution<br>(6) Triton™ X-100 surfactant described above | | | |

In Example 5, only example 5-14 with polymer P7 shows both (1) a drop in TEER at 4 hours permeation (which demonstrates good permeability) and good recovery of TEER 24 hours later (which demonstrates that the membranes recover from the treatment without permanent damage).

Example 6: Permeation Testing of formulations using example polymer P7

Permeation tests were performed as in Comparative Example 1. Two different batches of example polymer P7 were used, labeled P7-1 and P7-2. Results were as follows:

Permeation of SS

| Example: | 6-15 | 6-16 | 6-17 | 6-18 | 6-19 | 6-20 | 6-21 | 6-22 | 6-23 |
|---|---|---|---|---|---|---|---|---|---|
| Polymer: | P7-2 | P7-2 | P7-1 | P7-1 | P7-1 | P7-1 | P7-2 | P7-2 | P7-1 |
| Tissue(7) | S1 | S2 | S1 | S2 | S1 | S2 | S1 | S2 | - |
| Conc(1) | 0.5 | 0.5 | 0.2 | 0.2 | 0.2 | 0.2 | 0.2 | 0.2 | 0.02 |
| day | 1 | 1 | 2 | 2 | 3 | 3 | 3 | 3 | 3 |
| | | | | | | | | | |
| Remain(3) | 31.9 | 53.6 | 60.0 | 88.7 | 15.5 | 16.7 | 15.5 | 11.9 | 44.2 |

(continued)

| Example: | 6-15 | 6-16 | 6-17 | 6-18 | 6-19 | 6-20 | 6-21 | 6-22 | 6-23 |
|---|---|---|---|---|---|---|---|---|---|
| Perm(4) | 22.3 | 28.5 | 37.5 | 14.1 | 73.6 | 55.6 | 72.5 | 72.5 | 44.2 |
| Peff(5) | 2.6 | 3.3 | 4.3 | 1.6 | 8.5 | 6.4 | 8.4 | 8.4 | 5.1 |

(1) % weight of polymer on total weight of solution
(3) weight % of SS remaining in donor solution based on total SS
(4) weight % or SS permeated through tissue based on total SS
(5) units are $10^{-6}$ cm/sec
(7) Pairs of examples (such as 6-15 and 6-16) that are identical except for "tissue" are replicate examples performed on two tissue samples that were different from each other. Each example was performed on a separate individual tissue sample; therefore, for example, the tissue "S1" of 6-15 is not the same tissue sample as "S1" of 6-17.

Example 7: Permeation testing of various example polymers. Further permeation testing was conducted as in Comparative Example 1. Two batches of P7 were used: P7-1 and P7-2. Results were as follows:

Permeation of SS

| Example: | 7-24 | 7-25 | 7-26 | 7-27 | 7-28 | 7-29 |
|---|---|---|---|---|---|---|
| Polymer: | P1 | P2 | P3 | P4 | P5 | P6 |
| Conc(1) | 0.2 | 0.2 | 0.2 | 0.2 | 0.2 | 0.2 |
| | | | | | | |
| Remain(3) | 20.2 | 36.5 | 16.5 | 38.3 | 22.5 | 33.8 |
| Perm(4) | 56.6 | 44.2 | 39.5 | 40.4 | 34.9 | 23.1 |
| Peff(5) | 6.6 | 5.1 | 4.6 | 4.7 | 4.0 | 2.7 |

Permeation of SS

| Example: | 7-30 | 7-31 | 7-32 | C7-33 | C7-34 |
|---|---|---|---|---|---|
| Polymer: | P7-1 | P8 | P7-2 | Chitosan | none |
| Conc(1) | 0.2 | 0.02 | 0.2 | 0.2 | 0 |
| | | | | | |
| Remain(3) | 16.7 | 42.5 | 11.9 | 51.6 | 86.4 |
| Perm(4) | 55.6 | 37.9 | 72.5 | 32.4 | 1.7 |
| Peff(5) | 6.4 | 4.4 | 8.4 | 3.8 | 0.19 |

(1) % weight of polymer on total weight of solution
(2) number of replicate samples tested. Results shown are averages over the replicates
(3) weight % of SS remaining in donor solution based on total SS
(4) weight % or SS permeated through tissue based on total SS
(5) units are $10^{-6}$ cm/sec

All of the example polymers P1 through P8 show significant improvement to permeability over the control sample that has no polymer.

## Claims

1. An aqueous solution for use in a method of delivering a drug into the tissues and/or bloodstream of a living body by permeation through a mucosal surface in a nasal cavity, comprising:

   (a) a cationic polymer dissolved in water, wherein said cationic polymer comprises a hydrophobic quaternary ammonium group covalently attached to a hydroxyethyl cellulose polymer backbone; wherein the cationic polymer has a mole ratio of non-hydrophobic quaternary ammonium groups to hydrophobic quaternary ammonium groups of 0: 1; and
   (b) one or more physiologically active agents-

2. The aqueous solution for use according to rrF claim 1, wherein the amount of said cationic polymer is 0.01% to 10% by weight based on the weight of said aqueous solution.

3. The aqueous solution for use according to claim 1, wherein said cationic polymer has repeat units of structure II

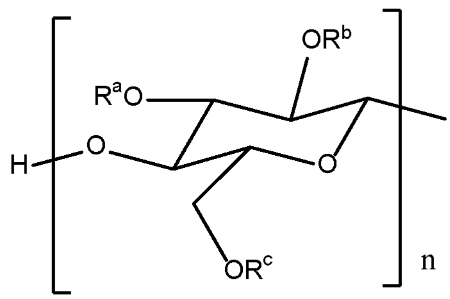

II

   wherein n is 10 or higher; $-R^a$, $-R^b$, and $-R^c$ is each H or $-[CH_2CH_2O]_x-R^1$, wherein each x is chosen from 0, 1, 2, 3, or 4; wherein $R^1$ is H or Structure III:

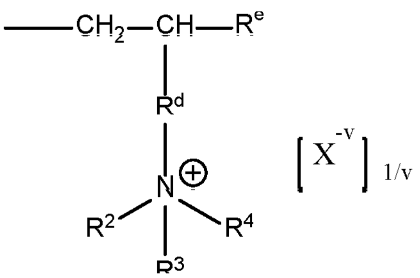

III

   wherein $-R^d-$ is a bivalent organic group, and $-R^e$ is either a hydrogen atom or a hydroxyl (OH) group; wherein at least one repeat unit has at least one of $-R^a$, $-R^b$, and $-R^c$ in which x is 1, 2, 3, or 4; wherein one or more of $-R^a$, $-R^b$, and $-R^c$ has the structure - $[CH_2CH_2O]_x-R^1$; wherein $-R^2$ and $-R^3$ is each an alkyl group with 3 or few carbon atoms;
   wherein $R^4$ is an alkyl group with 10 or more carbon atoms; and wherein X is an anion of valence v.

4. The aqueous solution for use according to ef claim 3, wherein $-R^d-$ is $-CH_2-$; $-R^e-$ is -OH; $-R^2$ and $-R^3$ is each methyl; and $-R^4$ is an alkyl group with 12 or more carbon atoms.

5. The aqueous solution for use according to any of claim 1 to claim 4, having a viscosity of 300 mPa-s or less when measured by steady shear viscometry using cone and plate at 10 sec-1 at 25°C.

## Patentansprüche

1. Wässrige Lösung zur Verwendung in einem Verfahren zum Abgeben eines Arzneimittels in die Gewebe und/oder den Blutkreislauf eines lebenden Körpers mittels Permeation durch eine Schleimhautoberfläche in einer Nasenhöhle, umfassend:

(a) ein kationisches Polymer, aufgelöst in Wasser, wobei das kationische Polymer eine hydrophobe quartäre Ammoniumgruppe umfasst, die kovalent an einen Hydroxyethylcellulose-Polymer-Backbone gebunden ist; wobei das kationische Polymer ein Molverhältnis von nichthydrophoben quartären Ammoniumgruppen zu hydrophoben quartären Ammoniumgruppen 0 : 1 beträgt; und
(b) ein oder mehrere physiologisch wirksame Mittel.

**2.** Wässrige Lösung zur Verwendung nach Anspruch 1, wobei die Menge des kationischen Polymers 0,01 Gew.-% bis 10 Gew.-%, bezogen auf das Gewicht der wässrigen Lösung, beträgt.

**3.** Wässrige Lösung zur Verwendung nach Anspruch 1, wobei das kationische Polymer Widerholeinheiten der Struktur II aufweist:

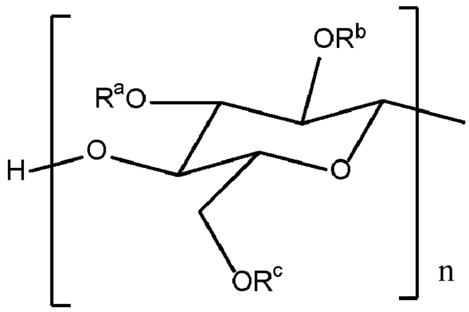

II

wobei n 10 oder höher ist; $-R^a$, $-R^b$ und $-R^c$ jeweils H oder $-[CH_2CH_2-O]_x-R^1$ sind, wobei x jeweils ausgewählt ist aus 0, 1, 2, 3 oder 4; wobei $R^1$ H oder Struktur III ist:

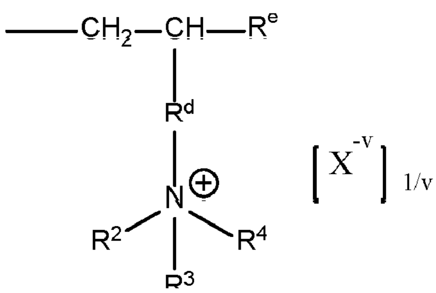

III

wobei $-R^d-$ eine zweiwertige organische Gruppe ist und $-R^e$ entweder ein Wasserstoffatom oder eine Hydroxyl(OH)-Gruppe ist; wobei mindestens eine Widerholeinheit mindestens eines von $-R^a$, $-R^b$ und $-R^c$ aufweist, wobei x 1, 2, 3 oder 4; wobei eines oder mehrere von $-R^a$, $-R^b$ und $-R^c$ die Struktur $-[CH_2CH_2O]_x-R^1$ aufweisen; wobei $-R^2$ und $-R^3$ jeweils eine Alkylgruppe mit 3 oder weniger Kohlenstoffatomen sind;
wobei $R^4$ eine Alkylgruppe mit 10 oder mehr Kohlenstoffatomen ist; und wobei X ein Anion mit Valenz v ist.

**4.** Wässrige Lösung zur Verwendung nach Anspruch 3, wobei $-R^d-$ $-CH_2-$ ist; $-R^e-$ -OH ist; $-R^2$ und $-R^3$ jeweils Methyl sind; und $-R^4$ eine Alkylgruppe mit 12 oder mehr Kohlenstoffatomen ist.

**5.** Wässrige Lösung zur Verwendung nach einem der Ansprüche 1 bis 4 mit einer Viskosität von 300 mPa-s oder weniger bei Messung mittels Steady-Scherviskosimetrie unter Verwendung von Kegel und Platte mit 10 s-1 bei 25 °C.

**Revendications**

**1.** Solution aqueuse pour utilisation dans un procédé d'administration d'un médicament dans les tissus et/ou la circulation sanguine d'un corps vivant par perméation à travers une surface muqueuse dans une cavité nasale, comprenant :

(a) un polymère cationique dissous dans l'eau, dans laquelle ledit polymère cationique comprend un groupe

ammonium quaternaire hydrophobe lié de façon covalente à un squelette d'hydroxyéthylcellulose ; dans laquelle le polymère cationique présente un rapport molaire des groupes ammonium quaternaire non hydrophobes aux groupes ammonium quaternaire hydrophobes de 0:1 ; et
(b) un ou plusieurs agents physiologiquement actifs.

**2.** Solution aqueuse pour utilisation selon la revendication 1, dans laquelle la quantité dudit polymère cationique est de 0,01 % à 10 % en poids sur la base du poids de ladite solution aqueuse.

**3.** Solution aqueuse pour utilisation selon la revendication 1, dans laquelle ledit polymère cationique comporte des motifs de répétition de structure II

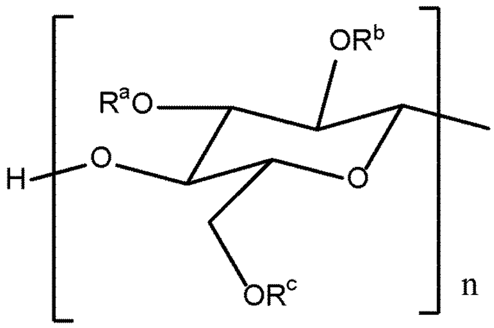

II

dans laquelle n est 10 ou plus ; -$R^a$, -$R^b$, et -$R^c$ sont chacun H ou - $[CH_2CH_2O]_x$-$R^1$, dans lequel chaque x est choisi parmi 0, 1, 2, 3 ou 4 ; dans lequel $R^1$ est H ou la structure III :

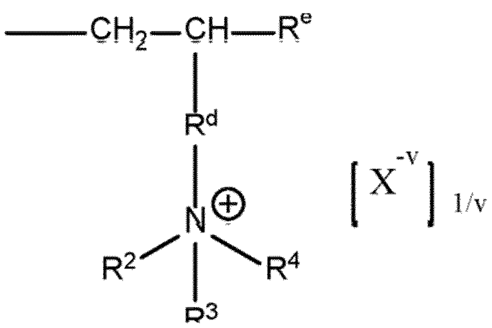

III

dans laquelle -$R^d$- est un groupe organique bivalent, et -$R^e$ est soit un atome d'hydrogène ou un groupe hydroxyle (OH) ; dans laquelle au moins un motif de répétition comporte au moins un parmi -$R^a$, -$R^b$ et -$R^c$, dans laquelle x est 1, 2, 3 ou 4 ; dans laquelle un ou plusieurs parmi -$R^a$, -$R^b$, et -$R^c$ ont la structure - $[CH_2CH_2O]_x$-$R^1$ ; dans laquelle -$R^2$ et -$R^3$ sont chacun un groupe alkyle avec 3 atomes de carbone ou moins ; dans laquelle $R^4$ est un groupe alkyle avec 10 atomes de carbone ou plus ; et dans laquelle X est un anion de valence v.

**4.** Solution aqueuse pour utilisation selon la revendication 3, dans laquelle -$R^d$- est -$CH_2$- ; -$R^e$- est -OH ; -$R^2$ et -$R^3$ sont chacun méthyle ; et -$R^4$ est un groupe alkyle avec 12 atomes de carbone ou plus.

**5.** Solution aqueuse pour utilisation selon l'une quelconque de la revendication 1 à la revendication 4, ayant une viscosité de 300 mPa-s ou moins lorsqu'elle est mesurée par viscosimétrie à cisaillement constant en utilisant un cône et une plaque à 10 $s^{-1}$ à 25 °C.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3472840 A
- WO 2015009799 A